# EUROPÄISCHE PATENTSCHRIFT

(11) **EP 3 253 317 B1**
(45) Veröffentlichungstag und Bekanntmachung des Hinweises auf die Patenterteilung: **05.12.2018**
(21) Anmeldenummer: 16703307.5
(22) Anmeldetag: 05.02.2016
(51) Int. Cl.: A61B 18/14

(54) **ENDOSKOPISCHES BEHANDLUNGSINSTRUMENT FÜR HOCHFREQUENZCHIRURGISCHE OPERATIONEN**
ENDOSCOPIC TREATMENT INSTRUMENT FOR HIGH-FREQUENCY SURGICAL PROCEDURES
INSTRUMENT DE TRAITEMENT ENDOSCOPIQUE POUR OPÉRATIONS CHIRURGICALES À HAUTE FRÉQUENCE

(30) Priorität: 05.02.2015 EP 15154030
(43) Veröffentlichungstag der Anmeldung: 13.12.2017
(73) Patentinhaber: Endox Feinwerktechnik GmbH, 72574 Bad Urach (DE); Farin, Günter, 72070 Tübingen (DE)
(72) Erfinder: FARIN, Günter, 72070 Tübingen (DE)
(74) Vertreter: Lohr, Jöstingmeier & Partner
(86) Internationale Anmeldenummer: PCT/EP2016/052555
(87) Internationale Veröffentlichungsnummer: WO 2016/124767

(56) Entgegenhaltungen:
- JP-A- 2007 275 625
- US-A1- 2005 131 424
- US-A1- 2005 261 675

## Beschreibung

### Technisches Gebiet

Die Erfindung betrifft Instrumente für hochfrequenzchirurgische (HF-chirurgische), endoskopisch kontrollierte Operationen, beispielsweise für die endoskopische Submukosa Resektion (ESR) oder Dissektion (ESD) pathologischer Mukosa-Areale im Magendarmtrakt.

### Stand der Technik

Aus pathologischen Mukosa-Arealen - den sog. Läsionen - der Mukosa des Magendarmtrakts können Karzinomen entstehen, die eine der weltweit meisten Todesfälle infolge Krebs sind. Werden diese Mukosa-Areale rechtzeitig vor Bildung von Karzinomen diagnostiziert und entsprechend der diesbezüglichen Anforderung der Onkologie in sano (d.h. bis ins angrenzende gesunde Gewebe) entfernt, kann einer Karzinombildung und insbesondere einer Metastasierung in andere Organe prophylaktisch vorgebeugt werden. Um die Anforderung der Onkologie zu erfüllen, muss das jeweilige pathologisch Mukosa-Areal mit einem ausreichenden horizontalen und vertikalen Sicherheitsabstand vom pathologische Mukosa-Areal im gesunden Gewebe entfernt werden. Um sicher prüfen zu können, ob die Anforderung der Onkologie erfüllt sind, fordert die Pathologie, dass die Entfernung pathologischer Mukosa-Areale en-bloc (d.h. in einem Stück) erfolgt. Je größer ein pathologisches Mukosa-Areal ist, desto schwieriger ist es, die o.g. Anforderungen der Onkologie und Pathologie zu erfüllen.

Heute stehen hierfür zwei endoskopische Verfahren zur Verfügung, nämlich die in Japan entwickelte "Endoskopische Mukosa Dissektion (ESD)" und die in Deutschland entwickelte "Endoskopische Submukosa Resektion (ESR)".

Vor Anwendung bzw. als Bestandteil der ESD sowie der ESR ist eine Inzision in die Mukosa und eine Umschneidung der Mukosa inklusive der darunter befindlichen Submukosa um die Läsion herum und möglichst bis nahe an die unter der Submukosa befindliche Muskularis propria heran erforderlich. Hierbei darf jedoch nicht in oder gar durch die Muskularis propria geschnitten werden. Da die Wand des Kolon (sog. Dickdarm) mit ca. 2 mm nicht dick, sondern sehr dünn ist, und da die Mukosa und Submukosa, wo die Tumore bzw. Karzinome meistens entstehen, zusammen nur ca. 1 mm dünn sind, ist die endoskopische Inzision und Umschneidung dieser Gewebeschichten bezüglich einer iatrogenen Perforation der Darmwand sehr riskant. Mit den bisher hierfür verfügbaren hochfrequenzchirurgischen (HF-chirurgischen) Instrumenten ist die Inzision und Umschneidung sehr schwierig, erfordert ausreichende Erfahrung und handwerkliches Geschick, und ist außerdem sehr zeitaufwendig.

Für die Inzision und Umschneidung der Mukosa stehen heute verschiedene endoskopisch anwendbare HF-chirurgische Instrumente zur Verfügung.

Aus der US 4,708,137 ist ein Instrument mit einstellbarer Nadellänge bekannt. Hier ist nur eine Schnittführung mit einem Endoskop möglich. Dabei besteht ein sehr hohes Perforationsrisiko.

Aus US 2005/0131424 ist ein endoskopisch anwendbares Instrument nach dem Oberbegriff des Anspruchs 1 bekannt.

### Darstellung der Erfindung

Der Erfindung liegt die Aufgabe zugrunde, ein HF-chirurgisches Instrument zum HF-chirurgischen Schneiden und/oder Koagulieren biologischer Gewebe zu entwickeln, insbesondere zur Inzision mit definierter und automatisch kontrollierter Inzisionstiefe, bei welchem sich die HF-chirurgische Schneidelektrode automatisch zum jeweiligen Zielgewebe, insbesondere senkrecht zu dessen Oberfläche ausrichtet, und das auch dann, wenn die Oberfläche des Zielgewebes, wie beispielsweise im Magendarmtrakt üblich, nicht plan, sondern konvex und/oder konkav geformt ist.

Diese Aufgabe wird durch eine Vorrichtung nach Anspruch 1 gelöst. Vorteilhafte Ausgestaltungen der Erfindung sind in den Unteransprüchen angegeben.

Ein endoskopisch anwendbares Instrument zum HF-chirurgischen Schneiden und/oder Koagulieren biologischer Gewebe und insbesondere zum HF-chirurgischen Umschneiden polypenartiger oder flacher Läsionen der Mukosa des Magendarmtrakts, umfasst einen flexiblen Katheter mit einem distalen und einem proximalen Ende, der durch einen Instrumentierkanal eines flexiblen Endoskops bewegt werden kann und einen Effektor mit einer HF-chirurgischen Schneid- und/oder Koagulationselektrode am distalen Ende des Katheters.

Bevorzugt ist ein HF-Stromkabel oder eine Anschlussvorrichtung für ein HF-Stromkabel am proximalen Ende des Katheters und eine HF-Stromleitung im Katheter vorgesehen, welche die HF-chirurgische Schneid- und/oder Koagulations-elektrode mit dem HF-Stromkabel bzw. mit der Anschlussvorrichtung für ein HF-Stromkabel verbindet.

Der Effektor umfasst eine an seiner Oberfläche mit einer Isolationsschicht elektrisch isolierte metallische Stütz- und/oder Gleitkufe mit einem distalen und einem proximalen Ende, die zwischen diesen beiden Enden eine Abwinkelung und/oder Biegung hat, derart, dass das distale Ende der Gleitkufe bzw. des Effektors einen Abstand von der Mittelachse des distalen Endabschnitts des Katheters hat, wobei die der Richtung der Abwinkelung und/oder Biegung abgewandte Seite des Effektors als Stütz- und/oder Gleitfläche nutzbar ist.

Weiterhin umfasst der Effektor bevorzugt eine elektrisch nicht isolierte HF-chirurgische Schneid- und/oder Koagulationselektrode an der elektrisch isolierten Stütz- und/oder Gleitfläche (10), die durch die Isolationsschicht hindurch mit der metallischen Stütz- und/oder Gleitkufe mechanisch und elektrisch leitfähig verbunden ist.

Weiterhin umfasst der Effektor bevorzugt einen Effektorschaft am proximalen Ende der metallischen Stütz- und/oder Gleitkufe, der in das distale Ende des Katheters hineinragt und dort um die Mittelachse (6) des distalen Endabschnitts des Katheters passiv drehbar gelagert ist.

Weiterhin umfasst der Effektor bevorzugt eine Arretiervorrichtung zur axialen Arretierung des Effektorschafts im Katheter, um unbeabsichtigte axiale Bewegungen des Effektorschafts im Katheter zu vermeiden, ohne jedoch die passive Drehbarkeit des Effektorschafts inklusive des Effektors um die Mittelachse zu behindern.

Weiterhin umfasst der Effektor bevorzugt eine rotationsflexible elektrisch leitfähige Verbindung zwischen der metallischen Stütz- und/oder Gleitkufe inklusive der HF-chirurgischen Schneid- und/oder Koagulationselektrode und einem HF-Stromkabel oder einer Vorrichtung zum Anschluss eines HF-Stromkabels am proximalen Ende des Katheters umfasst.

Eine bevorzugte Arretiervorrichtung hat im distalen Endabschnitt des Katheters einen distalen Anschlag und einen proximalen Anschlag, die voneinander beabstandet im Katheter befestigt sind. Diese beiden Anschläge haben je ein Loch, in welchem der Effektorschaft frei rotierbar gelagert ist. Weiterhin ist ein Anschlagelement zwischen den beiden Anschlägen am Effektorschaft befestigt, derart, dass der Effektor frei um die Mittelachse rotierbar, aber in seiner axialen Bewegung begrenzt gelagert ist, und dass die rotationsflexible elektrische Verbindung zwischen der metallischen Stütz- und/oder Gleitkufe inklusive der HF-chirurgischen Schneid- und/oder Koagulations-Elektrode und einem HF-Stromkabel oder einer Vorrichtung zum Anschluss eines HF-Stromkabels am proximalen Ende des Katheters einen rotationsflexiblen monofilen oder multifilen metallischer Draht umfasst

In einer weiteren Ausgestaltung hat die Arretiervorrichtung im distalen Endabschnitt des Katheters einen distalen Anschlag und einen proximalen Anschlag, die voneinander beabstandet im Katheter befestigt sind, wobei diese beiden Anschläge je ein Loch haben, worin der Effektorschaft frei rotierbar gelagert ist. weiterhin ist ein Anschlagelement zwischen den beiden Anschlägen am Effektorschaft befestigt, derart, dass der Effektor frei um die Mittelachse rotierbar, aber in seiner axialen Bewegung begrenzt gelagert ist. Weiterhin umfasst die rotationsflexible elektrische Verbindung zwischen der metallischen Stütz- und /oder Gleitkufe inklusive der HF-chirurgischen Schneid- und/oder Koagulations-Elektrode und einem HF-Stromkabel oder einer Vorrichtung zum Anschluss eines HF-Stromkabels am proximalen Ende des Katheters zwei flexible monofile oder multifile metallische Drähte, die im Katheter nebeneinander angeordnet sind und von denen einer mechanisch und elektrisch leitfähig nur mit dem Effektorschaft und hierdurch mit der Schneid- und/oder Koagulationselektrode, und der andere mechanisch und elektrisch nur mit dem HF-Stromkabel oder einer Vorrichtung zum Anschluss eines HF-Stromkabels am proximalen Ende des Katheters verbunden ist

In einer weiteren Ausgestaltung hat die Arretiervorrichtung im distalen Endabschnitt des Katheters einen Anschlag, der im Katheter befestigt ist, wobei dieser Anschlag ein Loch hat, in welchem der Effektorschaft frei rotierbar gelagert ist. Weiterhin ist ein erstes Anschlagelement proximal vom Anschlag und ein zweites Anschlagelement distal vom Anschlag am proximalen Ende des Effektorschafts befestigt, derart, dass der Effektor frei um die Mittelachse rotierbar, aber in seiner axialen Bewegung begrenzt gelagert ist. Die rotationsflexible elektrische Verbindung zwischen der metallischen Stütz- und/oder Gleitkufe inklusive der HF-chirurgischen Schneid- und/oder Koagulations-Elektrode und einem HF-Stromkabel oder einer Vorrichtung zum Anschluss eines HF-Stromkabels am proximalen Ende des Katheters umfasst einen rotationsflexiblen monofilen oder multifilen metallischer Draht.

In einer weiteren Ausgestaltung hat die Arretiervorrichtung im distalen Endabschnitt des Katheters einen Anschlag, der im Katheter befestigt ist, wobei dieser Anschlag ein Loch hat, in welchem der Effektorschaft frei rotierbar gelagert ist. weiterhin ist ein erstes Anschlagelement proximal vom Anschlag und ein zweites Anschlagelement distal vom Anschlag am proximalen Ende des Effektorschafts befestigt, derart, dass der Effektor frei um die Mittelachse rotierbar, aber in seiner axialen Bewegung begrenzt gelagert ist, und dass die rotationsflexible elektrische Verbindung zwischen der metallischen Stütz- und /oder Gleitkufe inklusive der HF-chirurgischen Schneid- und/oder Koagulations-Elektrode und einem HF-Stromkabel oder einer Vorrichtung zum Anschluss eines HF-Stromkabels am proximalen Ende des Katheters zwei flexible monofile oder multifile metallischer Drähte umfasst, die im Katheter nebeneinander angeordnet sind und von denen einer mechanisch und elektrisch leitfähig nur mit dem Effektorschaft und hierdurch mit der Schneid- und/oder Koagulationselektrode, und der andere mechanisch und elektrisch nur mit dem HF-Stromkabel oder einer Vorrichtung zum Anschluss eines HF-Stromkabels am proximalen Ende des Katheters verbunden ist

In einer weiteren Ausgestaltung ist das zweite Anschlagelement vor dem distalen Ende des Katheters auf dem Effektorschaft befestigt wobei der Außendurchmesser dieses Anschlagelements ist größer als der Durchmesser des Lochs im Anschlagelement oder größer als der Innendurchmesser des Katheters.

In einer weiteren Ausgestaltung umfasst die Arretiervorrichtung im distalen Endabschnitt des Katheters einen metallischen Anschlag, der im Katheter befestigt ist, wobei dieser Anschlag ein Loch hat, in welchem der Effektorschaft frei rotierbar gelagert ist. Weiterhin ist ein Anschlagelement distal vom Anschlag am Effektorschafts befestigt, und die rotationsflexible elektrische Verbindung zwischen der metallischen Stütz- und /oder Gleitkufe inklusive der HF-chirurgischen Schneid- und/oder Koagulations-Elektrode und einem HF-Stromkabel oder einer Vorrichtung zum Anschluss eines HF-Stromkabels am proximalen Ende des Katheters umfasst einen rotationsflexiblen monofilen oder multifilen metallischen Draht, dessen Länge so bemessen ist, dass der Effektorschaft nicht aus den Katheter herausgezogen werden kann.

In einer weiteren Ausgestaltung umfasst die Arretiervorrichtung ein am Effektorschaft befestigtes Anschlagelement, dessen Außendurchmesser größer als der Innendurchmesser des Katheters ist, wobei die rotationsflexible elektrische Verbindung zwischen der metallischen Stütz- und /oder Gleitkufe inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters einen rotationsflexiblen monofilen oder multifilen metallischen Draht umfasst, dessen Länge so bemessen ist, dass der Effektorschaft nicht aus den Katheter herausgezogen werden kann, aber derart, dass Effektor frei um die Mittelachse rotierbar ist.

In einer weiteren Ausgestaltung umfasst die Arretiervorrichtung im distalen Endabschnitt des Katheters einen elektrisch leitfähigen bzw. metallischen Anschlag, der im Katheter befestigt ist, wobei dieser Anschlag ein Loch hat, in welchem der im Anschlag befindliche Effektorschaft frei rotierbar gelagert ist. Weiterhin ist ein erstes, elektrisch leitfähiges und bevorzugt metallisches Anschlagelement proximal vom Anschlag und ein zweites elektrisch leitfähiges und bevorzugt metallisches Anschlagelement distal vom Anschlag am proximalen Ende des Effektorschafts befestigt, derart, dass der Effektor frei um die Mittelachse rotierbar, aber in seiner axialen Bewegung begrenzt gelagert ist. Der metallische Anschlag ist mit einem elektrisch leitfähigen Draht verbunden der den Anschlag mit einem HF-Stromkabel oder einer Vorrichtung zum Anschluss eines HF-Stromkabels am proximalen Ende des Katheters verbindet, wobei der proximale Endbereich des metallischen Effektorschafts nicht mit einer Isolationsschicht elektrisch isoliert ist, derart, dass dann, wenn der Effektor gegen Gewebe gedrückt wird, dieser Endbereich des Effektorschafts gegen den Anschlag gedrückt und ein elektrisch leitfähiger Kontakt zwischen diesen beiden Elementen entsteht.

In einer weiteren Ausgestaltung umfasst die metallische Gleitkufe aus einen an seiner Oberfläche elektrisch isolierten Draht. Dieser Draht ist vorteilhafterweise am distalen Ende der Gleitkufe derart gefaltet, dass die beiden gefalteten Teile des Drahts nebeneinander eine spangenartige Gleitkufe bilden.

In einer weiteren Ausgestaltung umfasst die metallische Gleitkufe einen an seiner Oberfläche elektrisch isolierten metallischen Blechstreifen oder einen kurzen metallischen Drahtstift.

Bevorzugt beträgt die Länge der Schneidelektrode (11) 1 mm bis 3 mm.

In einer weiteren Ausgestaltung ist am Ende der metallischen Schneidelektrode eine Verdickung mit einem Durchmesser vorhanden, der größer ist als der Durchmesser des metallischen Drahtstifts ist.

Ein wesentliches Merkmal dieser neuen Instrumente ist die freie Rotierbarkeit des Effektors am distalen Ende des Katheters, die dank der Form dieses Effektors die Schneid- und/oder Koagulationselektrode automatisch zum Zielgewebe ausrichtet wenn der Effektor mehr oder weniger fest gegen das Zielgewebe gedrückt wird.

Allerdings stört manchmal, dass der Effektor dann, wenn er von Zielgewebe abgehoben wurde, die Schneid- und/oder Koagulationselektrode ebenfalls automatisch in eine beliebige andere Richtung drehte. Dies wird verursacht durch den rotationsflexible Draht / Litzen zwischen Effektorschaft am distalen Ende des Katheters und HF-Stromkabel bzw. Einrichtung zum Anschluss eines HF-Stromkabels am proximalen Ende des Katheters. Alle alle flexiblen metallischen Drähte, und das gilt auch für die Rotationsflexibilität, möchte nach Verbiegung und/oder Verdrehung in ihre Ruhestellung zurück. Dies geschieht auch bei den hier verwendeten sehr dünnen und/oder langen Drähte oder Litzen.

Vorteilhafte, hier dargestellt Ausgestaltungen vermeiden jegliche Verdrehung des Drahtes bzw. Litze, indem sie gar nicht verdreht werden müssen.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt die erfindungswesentlichen Details eines erfindungsgemäßen Instruments
Figur 2 zeigt ein Ausführungsbeispiel der Arretiervorrichtung 14 sowie der rotationsflexiblen elektrisch leitfähigen Verbindung (15)
Figur 3 zeigt ein weiteres Ausführungsbeispiel der Arretiervorrichtung 14 sowie der rotationsflexiblen elektrisch leitfähigen Verbindung (15)
Figur 4 zeigt ein weiteres Ausführungsbeispiel der Arretiervorrichtung 14 sowie der rotationsflexiblen elektrisch leitfähigen Verbindung (15)
Figur 5 zeigt ein weiteres Ausführungsbeispiel der Arretiervorrichtung 14 sowie der rotationsflexiblen elektrisch leitfähigen Verbindung (15)
Figur 6 zeigt ein weiteres Ausführungsbeispiel der Arretiervorrichtung 14 sowie der rotationsflexiblen elektrisch leitfähigen Verbindung (15)
Figur 7 zeigt ein weiteres Ausführungsbeispiel der Arretiervorrichtung 14 sowie der rotationsflexiblen elektrisch leitfähigen Verbindung (15)
Figur 8 zeigt ein weiteres Ausführungsbeispiel der Arretiervorrichtung 14 sowie der rotationsflexiblen elektrisch leitfähigen Verbindung (15)
Figur 9 zeigt die automatische Ausrichtung des Effektors
Figur 10 zeigt das Umschneiden eines Gewebeareals
Figur 11 zeigt ein umschnittenes Gewebeareal vergrößert

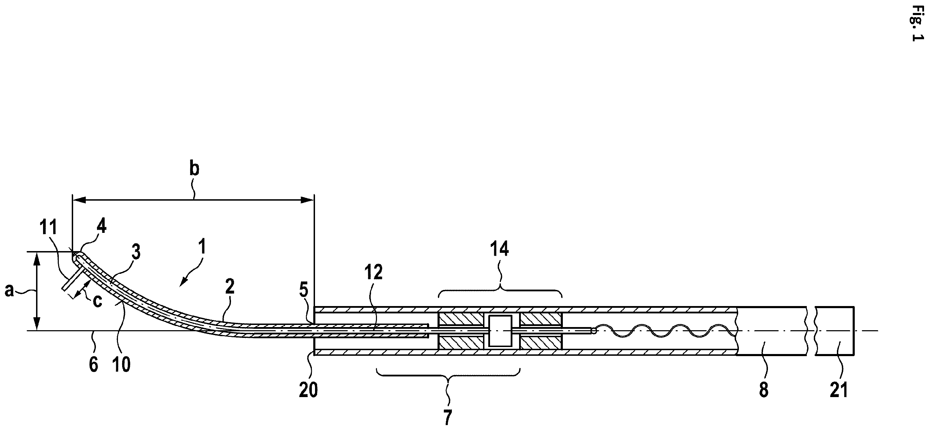

Ein in Fig. 1 schematisch dargestelltes erfindungsgemäßes Instrument zum HF-chirurgischen Schneiden und/oder Koagulieren biologischer Gewebe und insbesondere zum HF-chirurgischen Umschneiden polypenartiger oder flacher Läsionen der Mukosa des Magendarmtrakts, umfasst einen flexiblen Katheter (8) mit einem distalen (20) und einem proximalen (21) Ende, der durch einen Instrumentierkanal eines flexiblen Endoskops bewegt werden kann, einen Effektor (1) mit einer HF-chirurgischen Schneid- und/oder Koagulationselektrode am distalen Ende des Katheters, ein in Fig. 1 nicht gezeigtes HF-Stromkabel oder eine ebenfalls in Fig. 1 nicht gezeigte Anschlussvorrichtung für ein HF-Stromkabel am proximalen Ende (21) des Katheters und eine HF-Stromleitung (15) im Katheter, welche die HF-chirurgische Schneid- und/oder Koagulationselektrode mit dem HF-Stromkabel bzw. mit der Anschlussvorrichtung für ein HF-Stromkabel verbindet, und ist dadurch gekennzeichnet, dass der Effektor (1) folgendes umfasst:
- eine an ihrer Oberfläche mit einer Isolationsschicht (2) elektrisch isolierte metallische Stütz- und/oder Gleitkufe (3) mit einem distalen (4) und einem proximalen (5) Ende umfasst, die zwischen diesen beiden Enden (4, 5) eine Abwinkelung und/oder Biegung hat, derart, dass das distale Ende (4) der Gleitkufe (3) bzw. des Effektors (1) einen Abstand (a) von der Mittelachse (6) des distalen Endabschnitts (7) des Katheters (8) hat, wobei die der Richtung der Abwinkelung und/oder Biegung abgewandte Seite (10) des Effektors (1) als Stütz- und/oder Gleitfläche nutzbar ist,
- eine elektrisch nicht isolierte HF-chirurgische Schneid- und/oder Koagulationselektrode (11) an der elektrisch isolierten Stütz- und/oder Gleitfläche (10) hat, die durch die Isolationsschicht (2) hindurch mit der metallischen Stütz- und/oder Gleitkufe (3) mechanisch und elektrisch leitfähig verbunden ist,
- einen Effektorschaft (12) am proximalen Ende (5) der metallischen Stütz- und/oder Gleitkufe (3) hat, der in das distale Ende (7) des Katheters (8) hineinragt und dort um die Mittelachse (6) des distalen Endbereichs (7) des Katheters (8) passiv drehbar gelagert ist,
- eine Arretiervorrichtung (14) zur axialen Arretierung des Effektorschafts (12) im Katheter (8), um unbeabsichtigte axiale Bewegungen des Effektorschafts (12) im Katheter (8) zu vermeiden, ohne jedoch die passive Drehbarkeit des Effektorschafts (12) inklusive des Effektors (1) um die Mittelachse (6) zu behindern, und
- eine rotationsflexible elektrisch leitfähige Verbindung zwischen der metallischen Stütz- und/oder Gleitkufe (3) inklusive der HF-chirurgischen Schneid- und/oder Koagulations-elektrode (11) und einem HF-Stromkabel oder einer Vorrichtung zum Anschluss eines HF-Stromkabels am proximalen Ende (21) des Katheters.

Die an ihrer Oberfläche mit einer Isolationsschicht elektrisch isolierte metallische Stütz- und/oder Gleitkufe (3) des Effektors (1) kann verschieden Gestaltet und geformt sein, wobei es aber wichtig ist, dass sie, wie beispielsweise in Fig. 1 in seitlicher Ansicht dargestellt ist, zwischen ihren distalen und ihrem proximalen Ende eine Abwinkelung und/oder Biegung hat. So kann sie beispielsweise aus einem Draht oder einem Blechstreifen bestehen. Der Draht kann gestreckt oder derart gefaltet sein, dass die beiden gefalteten Teile des Drahts nebeneinander, beispielsweise wie bei einer Büroklammer, eine spangenartige Gleitkufe bilden.

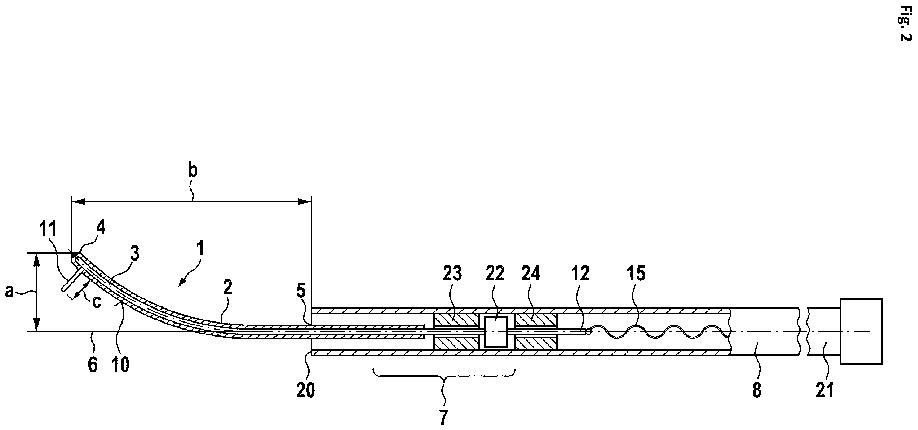

Anhand von Figur 2 wird im Folgenden ein Ausführungsbeispiel der Arretiervorrichtung (14) sowie der rotationsflexiblen elektrischen Verbindung (15) des anhand Fig. 1 beschriebene endoskopisch anwendbaren Instruments dargestellt und beschrieben. Die in Fig. 2 dargestellte Arretiervorrichtung (14) im distalen Endabschnitt (7) des Katheters (8) ist dadurch gekennzeichnet, dass sie einen distalen Anschlag (23) und einen proximalen Anschlag (24), die voneinander beabstandet im Katheter befestigt sind umfasst, und dass diese beiden Anschläge je ein Loch haben, in welchen der Effektorschaft (12) frei rotierbar gelagert ist, und dass ein Anschlagelement (22) zwischen den beiden Anschlägen (23, 24) am Effektorschaft befestigt ist, derart, dass der Effektor frei um die Mittelachse (6) rotierbar, aber in seiner axialen Bewegung begrenzt gelagert ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende (21) des Katheters (8) einen rotationsflexiblen monofilen oder multifilen metallischer Draht (15) umfasst.

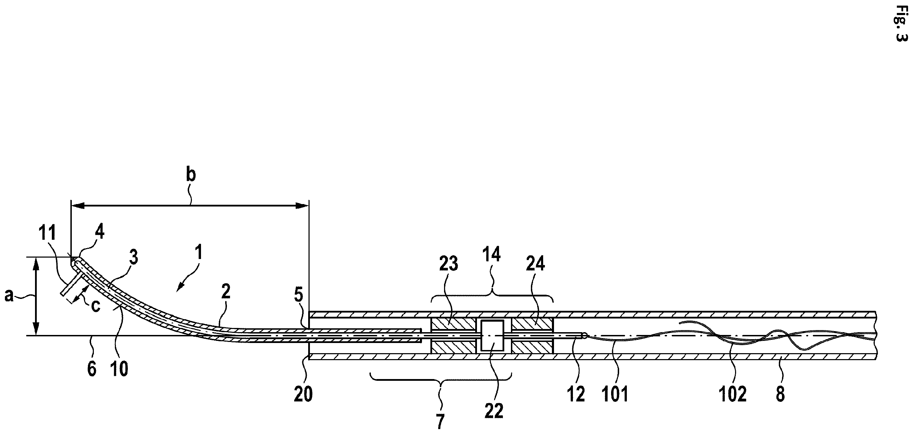

Anhand von Figur 3 wird im Folgenden ein weiteres Ausführungsbeispiel der Arretiervorrichtung (14) sowie der rotationsflexiblen elektrischen Verbindung (15) des anhand Fig. 1 beschriebene endoskopisch anwendbaren Instruments dargestellt und beschrieben. Die in Fig. 3 dargestellte Arretiervorrichtung (14) im distalen Endabschnitt (7) des Katheters (8) ist dadurch gekennzeichnet, dass sie einen distalen Anschlag (23) und einen proximalen Anschlag (24) umfasst, die voneinander beabstandet im Katheter befestigt sind, und dass diese beiden Anschläge je ein Loch haben, in welchen der Effektorschaft (12) frei rotierbar gelagert ist, und dass ein Anschlagelement (22) zwischen den beiden Anschlägen (23, 24) am Effektorschaft befestigt ist, derart, dass der Effektor frei um die Mittelachse (6) rotierbar, aber in seiner axialen Bewegung begrenzt gelagert ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) zwei flexible monofile oder multifile metallischer Drähte (101, 102) umfasst, die im Katheter nebeneinander angeordnet sind und von denen einer mechanisch und elektrisch leitfähig nur mit dem Effektorschaft und hierdurch mit der Schneid- und/oder Koagulationselektrode, und der andere mechanisch und elektrisch nur mit dem HF-Stromkabel oder einer Vorrichtung zum Anschluss eines HF-Stromkabels am proximalen Ende des Katheters (8) verbunden ist.

Diese Figur zeigt eine Ausführungsform, bei der der Draht bzw. die Litze nicht mehr verdreht werden müssen.

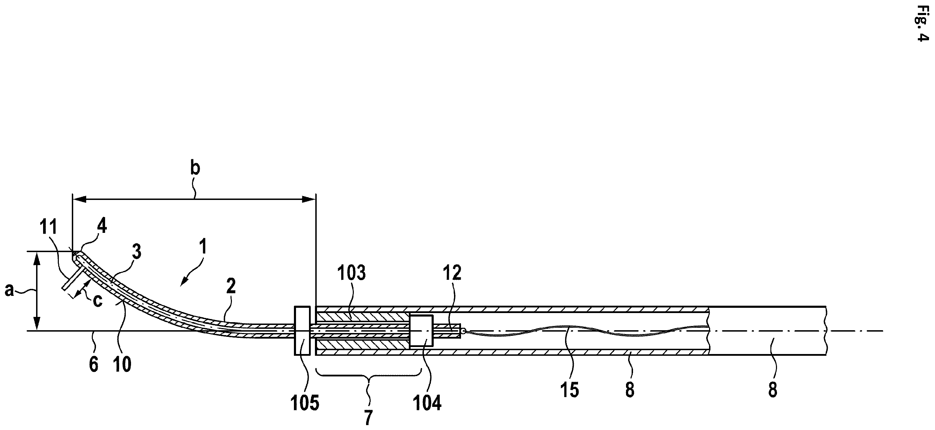

Anhand von Figur 4 wird im Folgenden ein weiteres Ausführungsbeispiel der Arretiervorrichtung sowie der rotationsflexiblen elektrischen Verbindung (15) des anhand Fig. 1 beschriebene endoskopisch anwendbaren Instruments dargestellt und beschrieben. Die in Fig. 4 dargestellt Arretiervorrichtung im distalen Endabschnitt (7) des Katheters (8) ist dadurch gekennzeichnet, dass sie einen Anschlag (103) umfasst, der im Katheter befestigt ist, und dass dieser Anschlag ein Loch hat, in welchem der Effektorschaft (12) frei rotierbar gelagert ist, und dass ein erstes Anschlagelement (104) proximal vom Anschlag (103) und ein zweites Anschlagelement (105) distal vom Anschlag (103) am proximalen Ende des Effektorschafts befestigt ist, derart, dass der Effektor frei um die Mittelachse (6) rotierbar, aber in seiner axialen Bewegung begrenzt gelagert ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) einen rotationsflexiblen monofilen oder multifilen metallischer Draht (15) umfasst.

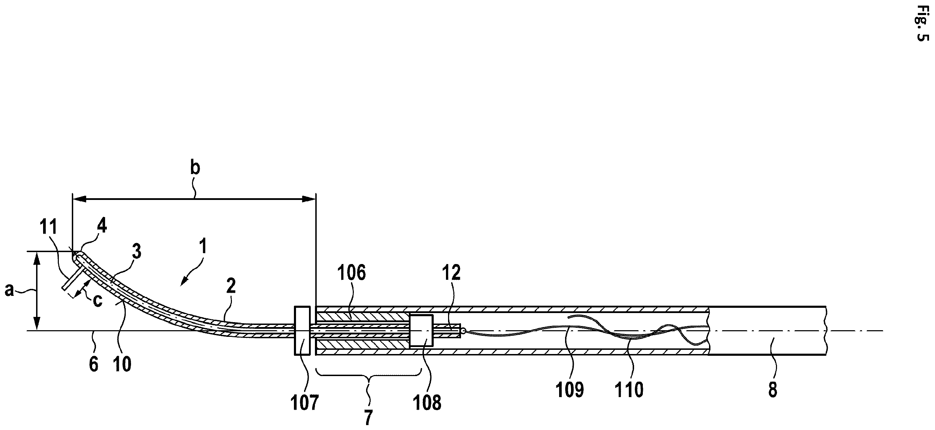

Anhand von Figur 5 wird im Folgenden ein weiteres Ausführungsbeispiel der Arretiervorrichtung (14) sowie der rotationsflexiblen elektrischen Verbindung (15) des anhand Fig. 1 beschriebene endoskopisch anwendbaren Instruments dargestellt und beschrieben. Die in Fig. 5 dargestellt Arretiervorrichtung im distalen Endabschnitt (7) des Katheters (8) ist dadurch gekennzeichnet, dass sie einen Anschlag (106) umfasst, der im Katheter befestigt ist, und dass dieser Anschlag ein Loch hat, in welchem der Effektorschaft (12) frei rotierbar gelagert ist, und dass ein erstes Anschlagelement (108) proximal vom Anschlag (106) und ein zweites Anschlagelement (107) distal vom Anschlag (106) am proximalen Ende des Effektorschafts befestigt ist, derart, dass der Effektor frei um die Mittelachse (6) rotierbar, aber in seiner axialen Bewegung begrenzt gelagert ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) zwei flexible monofile oder multifile metallischer Drähte (109, 110) umfasst, die im Katheter nebeneinander angeordnet sind und von denen einer mechanisch und elektrisch leitfähig nur mit dem Effektorschaft und hierdurch mit der Schneid- und/oder Koagulationselektrode, und der andere mechanisch und elektrisch nur mit dem HF-Stromkabel oder einer Vorrichtung zum Anschluss eines HF-Stromkabels am proximalen Ende des Katheters (8) verbunden ist.

Das zweite Anschlagelement (107) vor dem distalen Ende des Katheters kann auf dem Effektorschaft befestigt sein und der Außendurchmesser dieses Anschlagelements kann größer als der Durchmesser des Lochs im Anschlagelement oder größer als der Innendurchmesser des Katheters (8) sein.

Diese Figur zeigt eine weitere Ausführungsform, bei der der Draht bzw. die Litze nicht mehr verdreht werden müssen.

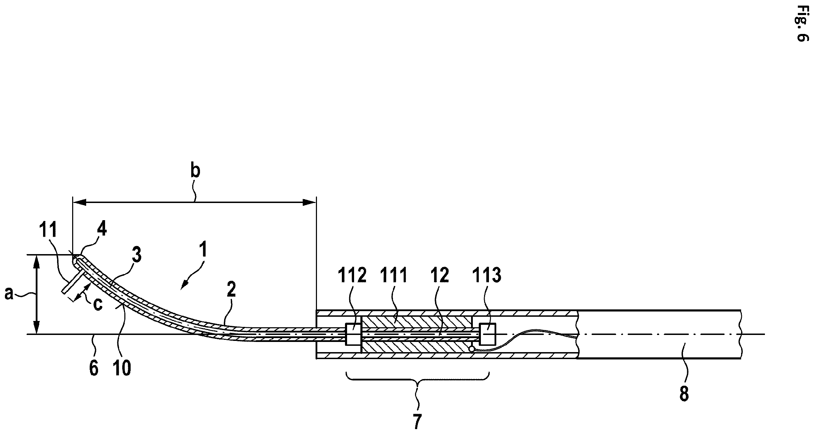

Anhand von Figur 6 wird im Folgenden ein weiteres Ausführungsbeispiel der Arretiervorrichtung (14) sowie der rotationsflexiblen elektrischen Verbindung (15) des anhand Fig. 1 beschriebene endoskopisch anwendbaren Instruments dargestellt und beschrieben. Die in Fig. 6 dargestellt Arretiervorrichtung im distalen Endabschnitt (7) des Katheters (8) ist dadurch gekennzeichnet, dass sie einen elektrisch leitfähigen bzw. metallischen Anschlag (111) umfasst, der im Katheter befestigt ist, und dass dieser Anschlag ein Loch hat, in welchem der im Anschlag (111) befindliche Effektorschaft (12) frei rotierbar gelagert ist, und dass ein erstes metallisches Anschlagelement (113) proximal vom Anschlag (111) und ein zweites metallisches Anschlagelement (112) distal vom Anschlag (111) am proximalen Ende des Effektorschafts befestigt ist, derart, dass der Effektor frei um die Mittelachse (6) rotierbar, aber in seiner axialen Bewegung begrenzt gelagert ist, und dass der metallische Anschlag (111) mit einem elektrisch leitfähigen Draht verbunden ist der den Anschlag mit einem HF-Stromkabel oder einer Vorrichtung zum Anschluss eines HF-Stromkabels am proximalen Ende des Katheters (8) verbindet, und dass der proximale Endbereich (12) des metallischen Effektorschafts nicht mit einer Isolationsschicht elektrisch isoliert ist, derart, dass dann, wenn der Effektor gegen Gewebe gedrückt wird, dieser Endbereich des Effektorschafts gegen den Anschlag gedrückt und ein elektrisch leitfähiger Kontakt zwischen diesen beiden Elementen entsteht.

Diese Figur zeigt eine weitere Ausführungsform, bei der der Draht bzw. die Litze gar nicht mehr verdreht werden müssen.

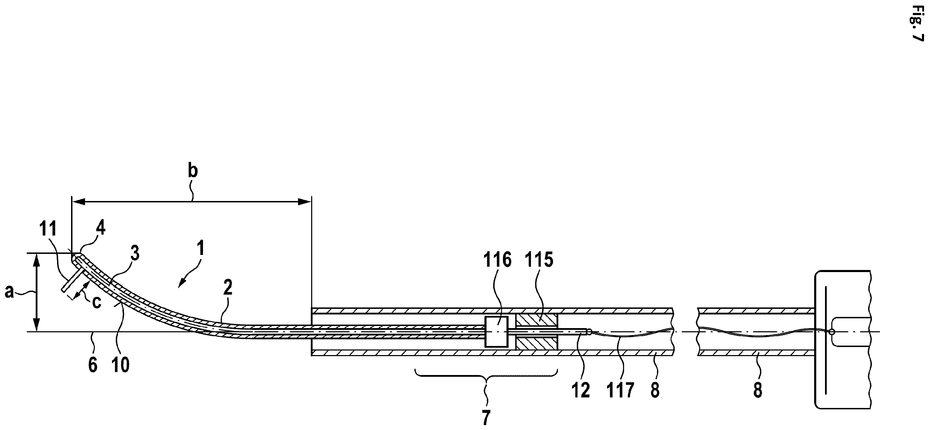

Anhand von Figur 7 wird im Folgenden ein weiteres Ausführungsbeispiel der Arretiervorrichtung (14) sowie der rotationsflexiblen elektrischen Verbindung (15) des anhand Fig. 1 beschriebene endoskopisch anwendbaren Instruments dargestellt und beschrieben. Die in Fig. 7 dargestellt Arretiervorrichtung im distalen Endabschnitt (7) des Katheters (8) ist dadurch gekennzeichnet, dass sie einen Anschlag (115) umfasst, der im Katheter befestigt ist, und dass dieser Anschlag ein Loch hat, in welchem der Effektorschaft (12) frei rotierbar gelagert ist, und dass ein Anschlagelement (116) distal vom Anschlag (115) am Effektorschafts befestigt ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) einen rotationsflexiblen monofilen oder multifilen metallischen Draht (117) umfasst, dessen Länge so bemessen ist, dass der Effektorschaft nicht aus den Katheter herausgezogen werden kann.

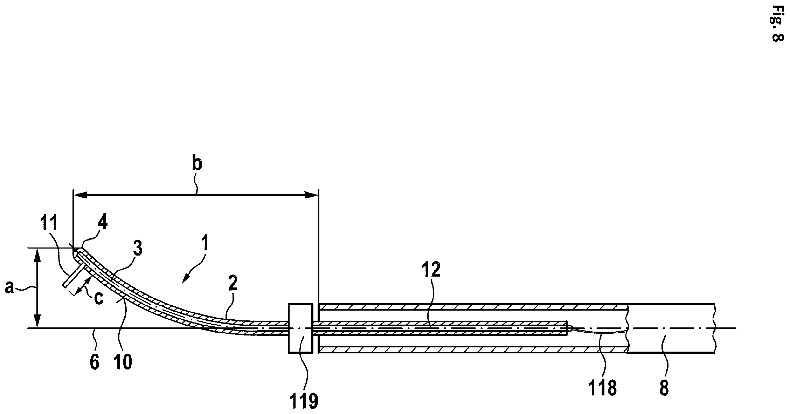

Anhand von Figur 8 wird im Folgenden ein weiteres Ausführungsbeispiel der Arretiervorrichtung (14) sowie der rotationsflexiblen elektrischen Verbindung (15) des anhand Fig. 1 beschriebene endoskopisch anwendbaren Instruments dargestellt und beschrieben. Die in Fig. 8 dargestellt Arretiervorrichtung ist dadurch gekennzeichnet, dass sie ein am Effektorschaft (12) befestigtes Anschlagelement (117) umfasst, dessen Außendurchmesser größer als der Innendurchmesser des Katheters ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) einen rotationsflexiblen monofilen oder multifilen metallischen Draht (118) umfasst, dessen Länge so bemessen ist, dass der Effektorschaft nicht aus den Katheter herausgezogen werden kann. bzw. derart, dass der Effektor frei um die Mittelachse (6) rotierbar, aber in seiner axialen Bewegung begrenzt gelagert ist.

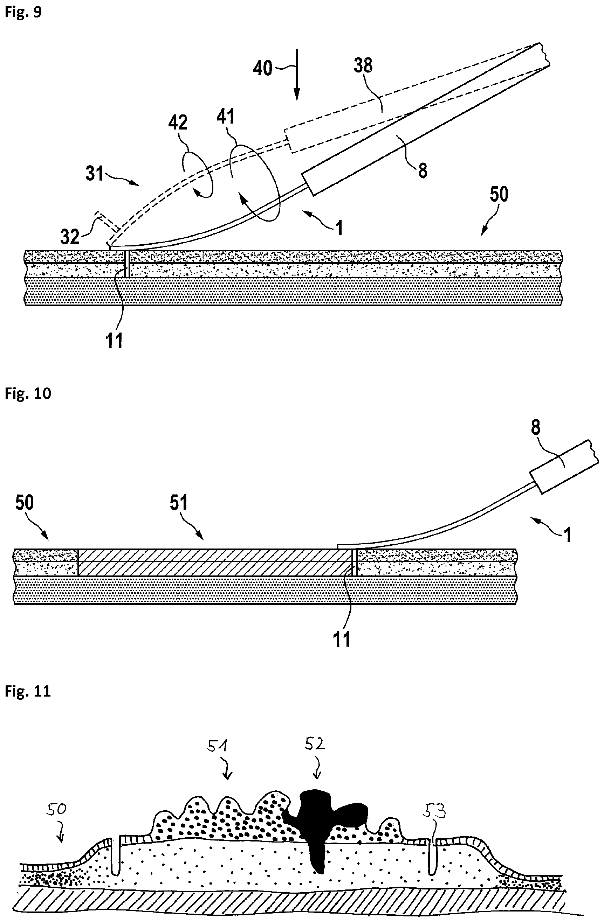

In Figur 9 ist die automatische Ausrichtung des Effektors dargestellt. Wird ein Katheter an ein Gewebe 50 herangeführt, so kann es sein, dass in einem ersten Zustand der Katheter 38 und der Effektor 31 falsch ausgerichtet sind, so dass die Schneid- und/oder Koagulationselektrode 32 vom Gewebe weg gerichtet ist. Wird nun eine Andruckkraft 40 durch den Katheter in Richtung des Gewebes ausgeübt, so dreht sich 42 der Effektor und das Instrument folgt mit einer linearen und/oder Drehbewegung 42 in Richtung des Gewebes. Nach dieser Ausrichtung kann die Elektrode 11 in das Gewebe eindringen und dieses schneiden.

Die Figur 10 zeigt, wie mit dem ausgerichteten Effektor ein Gewebeareal 51 umschnitten werden kann.

In der Figur 11 ist ein umschnittenes Gewebeareal 51 mit einem Tumor 52 vergrößert dargestellt.

### Bezugszeichenliste

- 1: Effektor
- 2: Isolationsschicht auf der Gleitkufe 3
- 3: Stütz- und Gleitkufe
- 4: distales Ende der Stütz- und Gleitkufe bzw. des Effektors
- 5: proximales Ende der Gleitkufe
- 6: Mittelachse des distalen Endabschnitts (7) des Katheters (8)
- 7: distaler Endabschnitts des Katheters (8)
- 8: Katheter
- 10: Stütz- und /oder Gleitfläche der Gleitkufe bzw. des Effektors
- 11: Schneid- und/oder Koagulationselektrode
- 12: Effektorschaft
- 14: Arretiervorrichtung
- 15: Rotationsflexible elektrisch leitfähige Verbindung zwischen der metallischen Stütz- und/oder Gleitkufe (3) inklusive der HF-chirurgischen Schneid- und/oder Koagulations-elektrode (11) und einem HF-Stromkabel oder einer Vorrichtung zum Anschluss eines HF-Stromkabels am proximalen Ende (21) des Katheters HF-Stromleitung im Katheter
- 16: Teil des gefalteten Drahts
- 20: distales Ende des Katheters (8)
- 21: proximales Ende des Katheters (8)
- 22: Anschlagelement am Effektorschaft (12)
- 23: distaler Anschlag
- 24: proximaler Anschlag
- 31: Effektor in einem ersten Zustand
- 32: Schneid- und/oder Koagulationselektrode in einem ersten Zustand
- 38: Katheter in einem ersten Zustand
- 40: Andruckkraft in Richtung Gewebe
- 41: Bewegung des Instruments
- 42: Bewegung des Effektors
- 50: Gewebe
- 51: zu entfernendes Gewebe
- 52: Tumor
- 101: metallischer Draht
- 102: metallischer Draht
- 103: Anschlag
- 104: Anschlagelement
- 105: Anschlagelement
- 106: Anschlag
- 107: Anschlagelement
- 108: Anschlagelement
- 109: Metallischer Draht
- 110: Metallischer Draht
- 111: Metallischer Anschlag
- 112: Metallisches Anschlagelement
- 113: Metallisches Anschlagelement
- 115: Anschlag
- 116: Anschlagelement
- 117: Rotationsflexibler metallischer Draht
- 118: Anschlagelement
- a: Abstand zwischen distalem Ende (4) der Gleitkufe bzw. des Effektors (1) und der Mittelachse (6) des distalen Endabschnitts (7) des Katheters (8)
- b: axiale Länge des Effektors (1)
- c: Abstand zwischen der Stütz- und /oder Gleitfläche (10) und dem Ende der Schneid- und/oder Koagulationselektrode (11)

## Patentansprüche

1. Endoskopisch anwendbares Instrument zum HF-chirurgischen Schneiden und/oder Koagulieren biologischer Gewebe und insbesondere zum HF-chirurgischen Umschneiden polypenartiger oder flacher Läsionen der Mukosa des Magendarmtrakts, umfassend einen flexiblen Katheter mit einem distalen und einem proximalen Ende, der durch einen Instrumentierkanal eines flexiblen Endoskops bewegt werden kann, einen Effektor mit einer HF-chirurgischen Schneid- und/oder Koagulationselektrode am distalen Ende des Katheters, ein HF-Stromkabel oder eine Anschlussvorrichtung für ein HF-Stromkabel am proximalen Ende des Katheters und eine HF-Stromleitung im Katheter, welche die HF-chirurgische Schneid- und/oder Koagulationselektrode mit dem HF-Stromkabel bzw. mit der Anschlussvorrichtung für ein HF-Stromkabel verbindet,
**dadurch gekennzeichnet, dass**
der Effektor (1)
- eine an ihrer Oberfläche mit einer Isolationsschicht (2) elektrisch isolierte metallische Stütz- und/oder Gleitkufe (3) mit einem distalen (4) und einem proximalen (5) Ende umfasst, die zwischen diesen beiden Enden (4, 5) eine Abwinkelung und/oder Biegung hat, derart, dass das distale Ende (4) der Gleitkufe (3) bzw. des Effektors (1) einen Abstand (a) von der Mittelachse (6) des distalen Endabschnitts (7) des Katheters (8) hat, wobei die der Richtung der Abwinkelung und/oder Biegung abgewandte Seite (10) des Effektors (1) als Stütz- und/oder Gleitfläche nutzbar ist,
- eine elektrisch nicht isolierte HF-chirurgische Schneid- und/oder Koagulationselektrode (11) an der elektrisch isolierten Stütz- und/oder Gleitfläche (10) hat, die durch die Isolationsschicht (2) hindurch mit der metallischen Stütz- und/oder Gleitkufe (3) mechanisch und elektrisch leitfähig verbunden ist,
- einen Effektorschaft (12) am proximalen Ende (5) der metallischen Stütz- und/oder Gleitkufe (3) hat, der in das distale Ende (20) des Katheters (8) hineinragt und dort um die Mittelachse (6) des distalen Endabschnitts (7) des Katheters (8) passiv drehbar gelagert ist,
- eine Arretiervorrichtung (14) zur axialen Arretierung des Effektorschafts (12) im Katheter (8), um unbeabsichtigte axiale Bewegungen des Effektorschafts (12) im Katheter (8) zu vermeiden, ohne jedoch die passive Drehbarkeit des Effektorschafts (12) inklusive des Effektors (1) um die Mittelachse (6) zu behindern, und
- eine rotationsflexible elektrisch leitfähige Verbindung zwischen der metallischen Stütz- und/oder Gleitkufe (3) inklusive der HF-chirurgischen Schneid- und/oder Koagulationselektrode (11) und einem HF-Stromkabel oder einer Vorrichtung zum Anschluss eines HF-Stromkabels am proximalen Ende (21) des Katheters umfasst.

2. Endoskopisch anwendbares Instrument nach Anspruch 1,
**dadurch gekennzeichnet, dass**
die Arretiervorrichtung (14) im distalen Endabschnitt (7) des Katheters (8) einen distalen Anschlag (23) und einen proximalen Anschlag (24) umfasst, die voneinander beabstandet im Katheter befestigt sind, und dass diese beiden Anschläge je ein Loch haben, in welchen der Effektorschaft (12) frei rotierbar gelagert ist, und dass ein Anschlagelement (22) zwischen den beiden Anschlägen (23, 24) am Effektorschaft befestigt ist, derart, dass der Effektor frei um die Mittelachse (6) rotierbar, aber in seiner axialen Bewegung begrenzt gelagert ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) einen rotationsflexiblen monofilen oder multifilen metallischer Draht (15) umfasst.

3. Endoskopisch anwendbares Instrument nach Anspruch 1,
**dadurch gekennzeichnet, dass**
die Arretiervorrichtung (14) im distalen Endabschnitt (7) des Katheters (8) einen distalen Anschlag (23) und einen proximalen Anschlag (24) umfasst, die voneinander beabstandet im Katheter befestigt sind, und dass diese beiden Anschläge je ein Loch haben, in welchen der Effektorschaft (12) frei rotierbar gelagert ist, und dass ein Anschlagelement (22) zwischen den beiden Anschlägen (23, 24) am Effektorschaft befestigt ist, derart, dass der Effektor frei um die Mittelachse (6) rotierbar, aber in seiner axialen Bewegung begrenzt gelagert ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) zwei flexible monofile oder multifile metallischer Drähte (101, 102) umfasst, die im Katheter nebeneinander angeordnet sind und von denen einer (101) mechanisch und elektrisch leitfähig nur mit dem Effektorschaft und hierdurch mit der Schneid- und/oder Koagulationselektrode, und der andere (102) mechanisch und elektrisch nur mit dem HF-Stromkabel oder einer Vorrichtung zum Anschluss eines HF-Stromkabels am proximalen Ende des Katheters (8) verbunden ist.

4. Endoskopisch anwendbares Instrument nach Anspruch 1,
**dadurch gekennzeichnet, dass**
die Arretiervorrichtung im distalen Endabschnitt (7) des Katheters (8) einen Anschlag (103) umfasst, der im Katheter befestigt ist, und dass dieser Anschlag ein Loch hat, in welchem der Effektorschaft (12) frei rotierbar gelagert ist, und dass ein erstes Anschlagelement (104) proximal vom Anschlag (103) und ein zweites Anschlagelement (105) distal vom Anschlag (103) am proximalen Ende des Effektorschafts befestigt ist, derart, dass der Effektor frei um die Mittelachse (6) rotierbar, aber in seiner axialen Bewegung begrenzt gelagert ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) einen rotationsflexiblen monofilen oder multifilen metallischer Draht (15) umfasst.

5. Endoskopisch anwendbares Instrument nach Anspruch 1,
**dadurch gekennzeichnet, dass**
die Arretiervorrichtung im distalen Endabschnitt (7) des Katheters (8) einen Anschlag (106) umfasst, der im Katheter befestigt ist, und dass dieser Anschlag ein Loch hat, in welchem der Effektorschaft (12) frei rotierbar gelagert ist, und dass ein erstes Anschlagelement (107) proximal vom Anschlag (106) und ein zweites Anschlagelement (108) distal vom Anschlag (106) am proximalen Ende des Effektorschafts befestigt ist, derart, dass der Effektor frei um die Mittelachse (6) rotierbar, aber in seiner axialen Bewegung begrenzt gelagert ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) zwei flexible monofile oder multifile metallischer Drähte (109, 110) umfasst, die im Katheter nebeneinander angeordnet sind und von denen einer mechanisch und elektrisch leitfähig nur mit dem Effektorschaft und hierdurch mit der Schneid- und/oder Koagulationselektrode, und der andere mechanisch und elektrisch nur mit dem HF-Stromkabel oder einer Vorrichtung zum Anschluss eines HF-Stromkabels am proximalen Ende des Katheters (8) verbunden ist.

6. Endoskopisch anwendbares Instrument nach wenigstens einem der Ansprüche 4 oder 5,
**dadurch gekennzeichnet, dass**
das zweite Anschlagelement vor dem distalen Ende des Katheters auf dem Effektorschaft befestigt ist und dass der Außendurchmesser dieses Anschlagelements größer als der Durchmesser des Lochs im Anschlagelement oder größer als der Innendurchmesser des Katheters (8) ist.

7. Endoskopisch anwendbares Instrument nach Anspruch 1,
**dadurch gekennzeichnet, dass**
die Arretiervorrichtung im distalen Endabschnitt (7) des Katheters (8) einen metallischen Anschlag (111) umfasst, der im Katheter befestigt ist, und dass dieser Anschlag ein Loch hat, in welchem der Effektorschaft (12) frei rotierbar gelagert ist, und dass ein Anschlagelement (112) distal vom Anschlag (111) am Effektorschafts befestigt ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) einen rotationsflexiblen monofilen oder multifilen metallischen Draht (114) umfasst, dessen Länge so bemessen ist, dass der Effektorschaft nicht aus den Katheter herausgezogen werden kann.

8. Endoskopisch anwendbares Instrument nach Anspruch 1,
**dadurch gekennzeichnet, dass**
die Arretiervorrichtung ein am Effektorschaft (114) befestigtes Anschlagelement (115) umfasst, dessen Außendurchmesser größer als der Innendurchmesser des Katheters ist, und dass die rotationsflexible elektrische Verbindung zwischen der metallischen Stütz- und /oder Gleitkufe (3) inklusive der HF-chirurgischen Schneid- und/oder Koagulations-Elektrode (11) und einem HF-Stromkabel oder einer Vorrichtung zum Anschluss eines HF-Stromkabels am proximalen Ende des Katheters (8) einen rotationsflexiblen monofilen oder multifilen metallischen Draht umfasst, dessen Länge so bemessen ist, dass der Effektorschaft nicht aus den Katheter herausgezogen werden kann, aber derart, dass Effektor frei um die Mittelachse (6) rotierbar ist.

9. Endoskopisch anwendbares Instrument nach Anspruch 1,
**dadurch gekennzeichnet, dass**
die Arretiervorrichtung im distalen Endabschnitt (7) des Katheters (8) einen elektrisch leitfähigen bzw. metallischen Anschlag (111) umfasst, der im Katheter befestigt ist, und dass dieser Anschlag ein Loch hat, in welchem der im Anschlag befindliche Effektorschaft (12) frei rotierbar gelagert ist, und dass ein erstes metallisches Anschlagelement (113) proximal vom Anschlag (111) und ein zweites metallisches Anschlagelement (112) distal vom Anschlag (111) am proximalen Ende des Effektorschafts befestigt ist, derart, dass der Effektor frei um die Mittelachse (6) rotierbar, aber in seiner axialen Bewegung begrenzt gelagert ist, und dass der metallische Anschlag (111) mit einem elektrisch leitfähigen Draht verbunden ist der den Anschlag mit einem HF-Stromkabel oder einer Vorrichtung zum Anschluss eines HF-Stromkabels am proximalen Ende des Katheters (8) verbindet, und dass der proximale Endbereich des metallischen Effektorschafts nicht mit einer Isolationsschicht elektrisch isoliert ist, derart, dass dann, wenn der Effektor gegen Gewebe gedrückt wird, dieser Endbereich des Effektorschafts gegen den Anschlag gedrückt und ein elektrisch leitfähiger Kontakt zwischen diesen beiden Elementen entsteht.

10. Endoskopisch anwendbares Instrument nach wenigstens einem der vorhergehenden Ansprüche,
**dadurch gekennzeichnet, dass**
die metallische Gleitkufe (3) aus einem an seiner Oberfläche elektrisch isolierten Draht besteht.

11. Endoskopisch anwendbares Instrument nach Anspruch 10,
**dadurch gekennzeichnet, dass**
dass der elektrisch isolierte Draht am distalen Ende (4) der Gleitkufe (3) derart gefaltet ist, dass die beiden gefalteten Teile (16, 17) des Drahts nebeneinander eine spangenartige Gleitkufe (18) bilden.

12. Endoskopisch anwendbares Instrument nach wenigstens einem der vorhergehenden Ansprüche,
**dadurch gekennzeichnet, dass**
die metallische Gleitkufe aus einem an seiner Oberfläche elektrisch isolierten metallischen Blechstreifen besteht.

13. Endoskopisch anwendbares Instrument nach wenigstens einem der vorhergehenden Ansprüche,
**dadurch gekennzeichnet, dass**
die Schneidelektrode (10) ein kurzer metallischer Drahtstift ist.

14. Endoskopisch anwendbares Instrument nach wenigstens einem der vorhergehenden Ansprüche,
**dadurch gekennzeichnet, dass**
die Länge der Schneidelektrode (11) 1 mm bis 3 mm beträgt.

15. Endoskopisch anwendbares Instrument nach Anspruch 13,
**dadurch gekennzeichnet, dass**
am Ende der metallischen Schneidelektrode (11) eine Verdickung (19) mit einem Durchmesser vorhanden ist, der größer ist als der Durchmesser dieses metallischen Drahtstifts ist.

## Claims

1. An endoscopically applicable instrument for RF surgical cutting and/or coagulation of biological tissues, and in particular for RF surgical circumferential cutting of polypoid or flat mucosal lesions of the gastrointestinal tract, comprising a flexible catheter having a distal end and a proximal end, which catheter can be moved through an instrumentation channel of a flexible endoscope, an effector with an RF surgical cutting and/or coagulation electrode at the distal end of the catheter, an RF power cable or connecting device for an RF power cable at the proximal end of the catheter, and an RF power supply line in the catheter which connects the RF surgical cutting and/or coagulation electrode with the RF power cable or with the connecting device for an RF power cable,
**characterized in that**
the effector (1) comprises
- a metallic support and/or sliding skid (3) being electrically insulated with an insulating layer (2) on its surface and having a distal (4) and a proximal end (5), said support or sliding skid (3) having an angling and/or bending between these two ends (4, 5), such that the distal end (4) of the sliding skid (3) or of the effector (1), respectively, has a distance (a) of the center axis (6) of the distal end portion (7) of the catheter (8), wherein the side (10) of the effector (1) facing away from the direction of the angling and/or bending is usable as a support and/or sliding surface,
- an electrically non-insulated RF surgical cutting and/or coagulation electrode (11) on the electrically insulated support and/or sliding surface (10) being mechanically and electrically conductively connected with the metallic support and/or sliding skid (3), and thereby passes through the insulating layer (2),
- an effector shaft (12) at the proximal end (5) of the metallic support and/or sliding skid (3), which effector shaft (12) projects into the distal end (20) of the catheter (8) and is there mounted passively rotatable about the center axis (6) of the distal end portion (7) of the catheter (8),
- a locking device (14) for axially locking the effector shaft (12) in the catheter (8) in order to avoid unintentional axial movements of the effector shaft (12) in the catheter (8), but without hindering the passive rotation of the effector shaft (12) including the effector (1) around the center axis (6), and
- a rotationally flexible, electrically conductive connection between the metallic support and/or sliding skid (3) including the RF surgical cutting and/or coagulation electrode (11) and an RF power cable or a device for connecting an RF power cable to the proximal end (21) of the catheter.

2. An endoscopically applicable instrument according to claim 1,
**characterized in that**
the locking device (14) comprises a distal stop (23) and a proximal stop (24) in the distal end portion (7) of the catheter (8), said stops (23, 24) being fixed spaced apart from each other in the catheter, and that these two stops each have a hole in which the effector shaft (12) is mounted freely rotatable, and that a stop element (22) between the two stops (23, 24) is fixed to the effector shaft, such that the effector is freely rotatable around the center axis (6), but is mounted limited in its axial movement, and **in that** the rotationally flexible electrical connection between the metallic support and/or sliding skid (3) including the RF surgical cutting and/or coagulation electrode (11) and an RF power cable or a device for connecting an RF power cable at the proximal end of the catheter (8), comprises a rotationally flexible monofilament or multifilament metal wire (15).

3. An endoscopically applicable instrument according to claim 1,
**characterized in that** the
locking device (14) comprises a distal stop (23) and a proximal stop (24) in the distal end portion (7) of the catheter (8), said stops (23, 24) being fixed spaced apart from each other in the catheter, and that these two stops each have a hole in which the effector shaft (12) is mounted freely rotatable, and that a stop element (22) between the two stops (23, 24) is fixed at the effector shaft, such that the effector is freely rotatable around the center axis (6), but is mounted limited in its axial movement, and **in that** the rotationally flexible electrical connection between the metallic support and/or sliding skid (3) including the RF surgical cutting and/or coagulation electrode (11) and an RF power cable or a device for connecting an RF power cable at the proximal end of the catheter (8), comprises two flexible monofilament or multifilament metallic wires (101, 102) which are arranged next to each other in the catheter, and one (101) of which is connected mechanically and electrically conductive only with the effector shaft and thereby with the cutting and/or coagulation electrode, and the other (102) is mechanically and electrically connected only with the RF power cable or a device for connecting an RF power cable at the proximal end of the catheter (8).

4. Endoscopically applicable instrument according to claim 1,
**characterized in that**
the locking device comprises a stop (103) in the distal end portion (7) of the catheter (8) which stop (103) is fixed in the catheter, and that said stop has a hole in which the effector shaft (12) is mounted freely rotatable, and that a first stop element (104) is fixed proximally to the stop (103) and a second stop element (105) is fixed distally from the stop (103) at the proximal end of the effector shaft, such that the effector is freely rotatable around the center axis (6), but is mounted limited in its axial movement, and that the rotationally flexible electrical connection between the metallic support and/or sliding skid (3) including the RF surgical cutting and/or coagulation electrode (11) and an RF power cable or a device for connecting an RF power cable at the proximal end of the catheter (8) comprises a rotationally flexible monofilament or multifilament metallic wire (15).

5. Endoscopically applicable instrument according to claim 1,
**characterized in that**
the locking device comprises a stop (106) in the distal end portion (7) of the catheter (8) which stop (106) is fixed in the catheter and that said stop has a hole in which the effector shaft (12) is mounted freely rotatable, and that a first stop element (107) is fixed proximal to the stop (106) and a second stop element (108) is fixed distal to the stop (106) at the proximal end of the effector shaft, such that the effector is freely rotatable around the center axis (6), but is mounted limited in its axial movement, and **in that** the rotationally flexible electrical connection between the metallic support and/or sliding skid (3) including the RF surgical cutting and/or coagulation electrode (11) and an RF power cable or a device for connecting an RF power cable at the proximal end of the catheter (8), comprises two flexible monofilament or multifilament metallic wires (109, 110) which are arranged next to each other in the catheter, and one of which is connected mechanically and electrically conductive only with the effector shaft and thereby with the cutting and/or coagulation electrode, and the other is mechanically and electrically connected only with the RF power cable or a device for connecting an RF power cable at the proximal end of the catheter (8).

6. Endoscopically applicable instrument according to at least one of claims 4 or 5,
**characterized in that**
the second stop element is fixed to the effector shaft in front of the distal end of the catheter and that the outer diameter of said stop element is greater than the diameter of the hole in the stop element or is greater than the inner diameter of the catheter (8).

7. Endoscopically applicable instrument according to claim 1,
**characterized in that**
the locking device comprises a metallic stop (111) in the distal end portion (7) of the catheter (8), said stop (111) being fixed in the catheter, and that said stop has a hole in which the effector shaft (12) is mounted freely rotatable, and that a stop element (112) is fixed distal of the stop (111) at the effector shaft, and that the rotationally flexible electrical connection between the metallic support and/or sliding skid (3) including the RF surgical cutting and/or coagulation electrode (11) and an RF power cable or a device for connecting an RF power cable at the proximal end of the catheter (8) comprises a rotationally flexible monofilament or multifilament metallic wire (114) whose length is dimensioned such that the effector shaft cannot be pulled out of the catheter.

8. Endoscopically applicable instrument according to claim 1,
**characterized in that**
the locking device comprises a stop element (115) fixed at the effector shaft (114), the outer diameter of which is greater than the inner diameter of the catheter, and that the rotationally flexible electrical connection between the metallic support and/or sliding skid (3) including the RF surgical cutting and/or coagulation electrode (11) and an RF power cable or a device for connecting an RF power cable at the proximal end of the catheter (8) comprises a rotationally flexible monofilament or multifilament metallic wire whose length is dimensioned such that the effector shaft cannot be withdrawn from the catheter, but such that effector is freely rotatable about the center axis (6).

9. An endoscopically applicable instrument according to claim 1,
**characterized in that**
the locking device comprises an electrically conductive or, respectively, metallic stop (111) in the distal end portion (7) of the catheter (8), which stop (111) is fixed in the catheter, and **in that** said stop has a hole, in which the effector shaft (12) being placed in the stop is mounted freely rotatable, and **in that** a first metallic stop element (113) is fixed proximally to the stop (111) and a second metallic stop element (112) is fixed distally to the stop (111) at the proximal end of the effector shaft, such that the effector is freely rotatable about the center axis (6), but is mounted limited in its axial movement, and that the metallic stop (111) is connected to an electrically conductive wire, which connects the stop with an RF power cable or a device for connecting an RF power cable at the proximal end of the catheter (8), and that the proximal end region of the metallic effector shaft is not electrically insulated with an insulating layer, such that when the effector is pressed against tissue, this end portion of the effector shaft is pressed against the stop and an electrically conductive contact is generated between these two elements.

10. Endoscopically applicable instrument according to at least one of the preceding claims,
**characterized in that**
the metallic sliding skid (3) consists of a wire which is electrically insulated on its surface.

11. Endoscopically applicable instrument according to claim 10,
**characterized in that**
the electrically insulated wire is folded at the distal end (4) of the sliding skid (3) such that the two folded parts (16, 17) of the wire next to each other form a brace-like sliding skid (18).

12. Endoscopically applicable instrument according to at least one of the preceding claims,
**characterized in that**
the metallic sliding skid consists of a metallic sheet metal strip which is electrically insulated on its surface.

13. Endoscopically applicable instrument according to at least one of the preceding claims,
**characterized in that**
the cutting electrode (10) is a short metallic wire pin.

14. Endoscopically applicable instrument according to at least one of the preceding claims,
**characterized in that**
the length of the cutting electrode (11) is 1 mm to 3 mm.

15. Endoscopically applicable instrument according to claim 13,
**characterized in that**
at the end of the metallic cutting electrode (11), there is a thickening (19) with a diameter that is greater than the diameter of said metallic wire pin.

## Revendications

1. Instrument utilisable par voie endoscopique pour la découpe et/ou la coagulation par chirurgie HF de tissus biologiques et en particulier pour l'excision par chirurgie HF de lésions polypoïdes ou planes de la muqueuse du tractus gastrointestinal, comprenant un cathéter flexible avec une extrémité distale et une extrémité proximale qui peut être déplacé dans un conduit d'instrumentation d'un endoscope flexible, un effecteur avec une électrode de coupe et/ou de coagulation pour chirurgie HF à l'extrémité distale du cathéter, un câble électrique HF ou un dispositif de connexion d'un câble électrique HF à l'extrémité proximale du cathéter et une ligne électrique HF dans le cathéter qui connecte l'électrode de coupe et/ou de coagulation pour chirurgie HF au câble électrique HF ou au dispositif de connexion d'un câble électrique HF,
**caractérisé en ce que** l'effecteur (1)
- comprend un patin d'appui et/ou de glissement (3) métallique isolé électriquement à la surface par une couche isolante (2), avec une extrémité distale (4) et une extrémité proximale (5), qui présente entre ces deux extrémités (4, 5) un coude et/ou une pliure de telle sorte que l'extrémité distale (4) du patin de glissement (3) ou de l'effecteur (1) présente une distance (a) par rapport à l'axe médian (6) de la partie d'extrémité distale (7) du cathéter (8), le côté (10) de l'effecteur (1) tourné à l'opposé du coude et/ou de la pliure pouvant être utilisé comme surface d'appui et/ou de glissement,
- possède une électrode de coupe et/ou de coagulation pour chirurgie HF (11) non isolée électriquement sur la surface d'appui et/ou de glissement (10) isolée électriquement, qui est raccordée de façon mécaniquement et électriquement conductrice au patin d'appui et/ou de glissement métallique (3) à travers la couche isolante (2),
- possède, à l'extrémité proximale (5) du patin d'appui et/ou de glissement métallique (3), une tige d'effecteur (12) qui dépasse dans l'extrémité distale (20) du cathéter (8) et y est supportée avec possibilité de rotation passive autour de l'axe médian (6) de la partie d'extrémité distale (7) du cathéter (8),
- possède un dispositif de blocage (14) pour le blocage axial de la tige d'effecteur (12) dans le cathéter (8) afin d'éviter les mouvements axiaux involontaires de la tige d'effecteur (12) dans le cathéter (8), sans toutefois gêner la possibilité de rotation passive de la tige d'effecteur (12) avec l'effecteur (1) autour de l'axe médian (6), et
- comprend une liaison flexible en rotation et électriquement conductrice entre le patin d'appui et/ou de glissement métallique (3) avec l'électrode de coupe et/ou de coagulation pour chirurgie HF (11) et un câble électrique HF ou un dispositif de connexion d'un câble électrique HF à l'extrémité proximale (21) du cathéter.

2. Instrument utilisable par voie endoscopique selon la revendication 1, **caractérisé en ce que** le dispositif de blocage (14) dans la partie d'extrémité distale (7) du cathéter (8) comprend une butée distale (23) et une butée proximale (24) qui sont fixées dans le cathéter à distance l'une de l'autre, et **en ce que** ces deux butées possèdent chacune un trou dans lequel la tige d'effecteur (12) est supportée avec la possibilité de tourner librement, et **en ce qu'**un élément de butée (22) est fixé entre les deux butées (23, 24) sur la tige d'effecteur, de telle sorte que l'effecteur peut tourner librement autour de l'axe médian (6) mais est supporté avec une possibilité de mouvement axial limité, et **en ce que** la connexion électrique flexible en rotation entre le patin d'appui et/ou de glissement métallique (3) muni de l'électrode de coupe et/ou de coagulation pour chirurgie HF (11) et un câble électrique HF ou un dispositif de connexion d'un câble électrique HF à l'extrémité proximale du cathéter (8) comprend un fil métallique (15) monofilament ou multifilament flexible en rotation.

3. Instrument utilisable par voie endoscopique selon la revendication 1, **caractérisé en ce que** le dispositif de blocage (14) dans la partie d'extrémité distale (7) du cathéter (8) comprend une butée distale (23) et une butée proximale (24) qui sont fixées dans le cathéter à distance l'une de l'autre, et **en ce que** ces deux butées possèdent chacune un trou dans lequel la tige d'effecteur (12) est supportée avec la possibilité de tourner librement, et **en ce qu'**un élément de butée (22) est fixée entre les deux butées (23, 24) sur la tige d'effecteur, de telle sorte que l'effecteur peut tourner librement autour de l'axe médian (6) mais est supporté avec une possibilité de mouvement axial limité, et **en ce que** la connexion électrique flexible en rotation entre le patin d'appui et/ou de glissement métallique (3) muni de l'électrode de coupe et/ou de coagulation pour chirurgie HF (11) et un câble électrique HF ou un dispositif de connexion d'un câble électrique HF à l'extrémité proximale du cathéter (8) comprend deux fils métalliques (101, 102) monofilament ou multifilament flexibles en rotation qui sont disposés l'un à côté de l'autre dans le cathéter et dont l'un (101) est relié de façon mécaniquement et électriquement conductrice uniquement à la tige d'effecteur et, à travers celle-ci, à l'électrode de coupe et/ou de coagulation, et l'autre (102) est relié de façon mécaniquement et électriquement conductrice uniquement au câble électrique HF ou à un dispositif de connexion d'un câble électrique HF à l'extrémité proximale du cathéter (8).

4. Instrument utilisable par voie endoscopique selon la revendication 1, **caractérisé en ce que** le dispositif de blocage dans la partie d'extrémité distale (7) du cathéter (8) comprend une butée (103) qui est fixée dans le cathéter et **en ce que** cette butée possède un trou dans lequel la tige d'effecteur (12) est supportée avec la possibilité de tourner librement, et **en ce qu'**un premier élément de butée (104) est fixé sur l'extrémité proximale de la tige d'effecteur du côté proximal de la butée (103) et un deuxième élément de butée (105) du côté distal de la butée (103), de telle sorte que l'effecteur peut tourner librement autour de l'axe médian (6) mais est supporté avec une possibilité de mouvement axial limité, et **en ce que** la connexion électrique flexible en rotation entre le patin d'appui et/ou de glissement métallique (3) muni de l'électrode de coupe et/ou de coagulation pour chirurgie HF (11) et un câble électrique HF ou un dispositif de connexion d'un câble électrique HF à l'extrémité proximale du cathéter (8) comprend un fil métallique (15) monofilament ou multifilament flexible en rotation.

5. Instrument utilisable par voie endoscopique selon la revendication 1, **caractérisé en ce que** le dispositif de blocage dans la partie d'extrémité distale (7) du cathéter (8) comprend une butée (106) qui est fixée dans le cathéter et **en ce que** cette butée possède un trou dans lequel la tige d'effecteur (12) est supportée avec la possibilité de tourner librement, et **en ce qu'**un premier élément de butée (107) est fixé sur l'extrémité proximale de la tige d'effecteur du côté proximal de la butée (106) et un deuxième élément de buté (108) du côté distal de la butée (106) de telle sorte que l'effecteur peut tourner librement autour de l'axe médian (6) mais est supporté avec une possibilité de mouvement axial limité, et **en ce que** la connexion électrique flexible en rotation entre le patin d'appui et de glissement métallique (3) muni de l'électrode de coupe et/ou de coagulation pour chirurgie HF (11) et un câble électrique HF ou un dispositif de connexion d'un câble électrique HF à l'extrémité proximale du cathéter (8) comprend deux fils métalliques (109, 110) monofilament ou multifilament flexibles en rotation qui sont disposés l'un à côté de l'autre dans le cathéter et dont l'un est relié de façon mécaniquement et électriquement conductrice uniquement à la tige d'effecteur et, à travers celle-ci, à l'électrode de coupe et/ou de coagulation, et l'autre est relié de façon mécaniquement et électriquement conductrice uniquement au câble électrique HF ou à un dispositif de connexion d'un câble électrique HF à l'extrémité proximale du cathéter (8).

6. Instrument utilisable par voie endoscopique selon l'une au moins des revendications 4 ou 5, **caractérisé en ce que** le deuxième élément de butée sur la tige d'effecteur est fixé avant l'extrémité distale du cathéter et **en ce que** le diamètre extérieur de cet élément de butée est plus grand que le diamètre du trou de l'élément de butée ou plus grand que le diamètre intérieur du cathéter (8).

7. Instrument utilisable par voie endoscopique selon la revendication 1, **caractérisé en ce que** le dispositif de blocage dans la partie d'extrémité distale (7) du cathéter (8) comprend une butée métallique (111) qui est fixée dans le cathéter et **en ce que** cette butée possède un trou dans lequel la tige d'effecteur (12) peut tourner librement, et **en ce qu'**un élément de butée (112) est fixé du côté distal de la butée (111) sur la tige d'effecteur, et **en ce que** la connexion électrique flexible en rotation entre le patin d'appui et/ou de glissement métallique (3) muni de l'électrode de coupe et/ou de coagulation pour chirurgie HF (11) et un câble électrique HF ou un dispositif de connexion d'un câble électrique HF à l'extrémité proximale du cathéter (8) comprend un fil métallique (114) monofilament ou multifilament flexible en rotation dont la longueur est dimensionnée de telle façon que la tige d'effecteur ne puisse pas être retirée du cathéter.

8. Instrument utilisable par voie endoscopique selon la revendication 1, **caractérisé en ce que** le dispositif de blocage comprend un élément de butée (115) fixé sur la tige d'effecteur (114) et dont le diamètre extérieur est plus grand que le diamètre intérieur du cathéter, et **en ce que** la connexion électrique flexible en rotation entre le patin d'appui et/ou de glissement métallique (3) muni de l'électrode de coupe et/ou de coagulation pour chirurgie HF (11) et un câble électrique HF ou un dispositif de connexion d'un câble électrique HF à l'extrémité proximale du cathéter (8) comprend un fil métallique monofilament ou multifilament flexible en rotation dont la longueur est dimensionnée de telle façon que la tige d'effecteur ne puisse pas être retirée du cathéter mais que l'effecteur puisse tourner librement autour de l'axe médian (6).

9. Instrument utilisable par voie endoscopique selon la revendication 1, **caractérisé en ce que** le dispositif de blocage dans partie d'extrémité distale (7) du cathéter (8) comprend une butée conductrice électrique ou métallique (111) qui est fixée dans le cathéter et **en ce que** cette butée possède un trou dans lequel la tige d'effecteur (12) qui se trouve dans la butée est supportée avec la possibilité de tourner librement, et **en ce qu'**un premier élément de butée métallique (113) est fixé sur l'extrémité proximale de la tige d'effecteur du côté proximal de la butée (111) et un deuxième élément de butée métallique (112) du côté distal de la butée (111), de telle sorte que l'effecteur peut tourner librement autour de l'axe médian (6) mais est supporté avec une possibilité de mouvement axial limité, et **en ce que** la butée métallique (111) est reliée avec un fil métallique électriquement conducteur qui relie la butée à un câble électrique HF ou à un dispositif de connexion d'un câble électrique HF à l'extrémité proximale du cathéter (8), et **en ce que** la partie d'extrémité proximale de la tige d'effecteur métallique n'est pas isolée électriquement par une couche isolante, de sorte que lorsque l'effecteur est appuyé contre des tissus, cette partie d'extrémité de la tige d'effecteur est appuyée contre la butée et un contact conducteur électrique s'établit entre ces deux éléments.

10. Instrument utilisable par voie endoscopique selon une au moins des revendications précédentes, **caractérisé en ce que** le patin de glissement métallique (3) est formé d'un fil métallique isolé électriquement à sa surface.

11. Instrument utilisable par voie endoscopique selon la revendication 10, **caractérisé en ce que** le fil métallique isolé électriquement est replié à l'extrémité distale (4) du patin de glissement (3) de telle façon que les deux parties repliées (16, 17) du fil métallique forment l'une à côté de l'autre un patin de glissement (18) en forme de boucle.

12. Instrument utilisable par voie endoscopique selon une au moins des revendications précédentes, **caractérisé en ce que** le patin de glissement métallique se compose d'une bande de tôle métallique isolée électriquement à sa surface.

13. Instrument utilisable par voie endoscopique selon une au moins des revendications précédentes, **caractérisé en ce que** l'électrode de coupe (10) est une courte tige en fil métallique.

14. Instrument utilisable par voie endoscopique selon une au moins des revendications précédentes, **caractérisé en ce que** la longueur de l'électrode de coupe (11) est de 1 mm à 3 mm.

15. Instrument utilisable par voie endoscopique selon la revendication 13, **caractérisé en ce qu'**est formé à l'extrémité de l'électrode de coupe métallique (11) un épaississement (19) dont le diamètre est plus grand que le diamètre de cette tige de fil métallique.
